(19) Europäisches Patentamt

**European Patent Office**

**Office européen des brevets**

(11) Numéro de publication: **0 377 091 B1**

(12) **FASCICULE DE BREVET EUROPEEN**

(45) Date de publication de fascicule du brevet: **14.04.93** (51) Int. Cl.⁵: **A61K 7/48**

(21) Numéro de dépôt: **89119479.7**

(22) Date de dépôt: **20.10.89**

(54) **Préparation cosmétique contenant du chitosane.**

(30) Priorité: **28.11.88 CH 4418/88**

(43) Date de publication de la demande:
**11.07.90 Bulletin 90/28**

(45) Mention de la délivrance du brevet:
**14.04.93 Bulletin 93/15**

(84) Etats contractants désignés:
**AT BE CH DE ES FR GB GR IT LI LU NL SE**

(56) Documents cités:
**EP-A- 0 161 212**

**PATENT ABSTRACTS OF JAPAN, vol. 11, no. 290 (C-447)[2737], 18 septembre 1987, page 70 C 447**

**PATENT ABSTRACTS OF JAPAN, vol. 13, no. 115 (C-578)[3463], 20 mars 1989, page 131 C 578**

(73) Titulaire: **SOCIETE DES PRODUITS NESTLE S.A.**
**Case postale 353**
**CH-1800 Vevey(CH)**

(72) Inventeur: **Leuba, Jean-Louis**

**CH-1034 Boussens(CH)**
Inventeur: **Link, Harriet**
**Boulevard St-Martin 16**
**CH-1800 Vevey(CH)**
Inventeur: **Stoessel, Peter**
**Zentenarweg 2**
**CH-5000 Aarau(CH)**
Inventeur: **Viret, Jean-Louis**
**La Bruyère A Fontanivent**
**CH-1817 Brent(CH)**

## Description

La présente invention a trait à une préparation cosmétique contenant du chitosane.

La littérature contient de nombreux exemples d'utilisation du chitosane.

Le chitosane peut être utilisé dans le traitement des brûlures. Par immersion du corps brûlé dans une solution d'acétate de chitosane, on forme à la surface de la plaie un film résistant et perméable à l'oxygène, qui va faciliter la guérison.

Le chitosane est aussi connu pour posséder, sous forme de solution hydrochlorée, une bonne activité antiinfectieuse envers les bactéries, comme le montre le brevet JP 82.137930.

Le brevet JP 87.083877 mentionne l'utilisation de chitosane, de préférence dégradé par une chitonase, comme agent conservateur de divers produits.

Dans le domaine cosmétique, le chitosane est surtout connu pour être introduit dans des shampooings et des produits capillaires afin d'assurer la mise en forme et la fixation des cheveux, comme dans le brevet DE 2627419.

Le chitosane peut aussi permettre l'amélioration des propriétés lavantes et massantes d'un composé appliqué sur la peau, comme le décrit le brevet JP 86.210014.

D'une manière générale les préparations cosmétiques emballées stérilement se conservent assez bien. Toutefois, elles sont soumises à des risques de contamination tout au long de leur emploi.

Lors de l'ouverture du récipient contenant la préparation, les microorganismes contenus dans l'air ambiant peuvent la contaminer.

Puis lors de l'utilisation de la préparation, les microorganismes se trouvant à la surface de la peau peuvent aussi la contaminer. Ces contaminations peuvent entraîner une dégradation de la préparation.

La présente invention a pour but de pallier les inconvénients de l'art antérieur et de proposer une préparation cosmétique ayant de bonnes propriétés de conservation, contenant du chitosane comme agent de conservation.

A cet effet le chitosane selon la présente invention est choisi parmi ses polymères ayant un poids moléculaire compris entre 3000 et 700 000.

Un avantage de cette invention est d'utiliser comme agent de conservation un produit naturel.

Un autre avantage est d'utiliser comme agent de conservation un produit abondant et bon marché.

Un autre avantage est de n'introduire qu'une faible quantité de chitosane dans la préparation cosmétique.

Un autre avantage est d'employer un produit vis-à-vis duquel les microorganismes n'ont pas d'accoutumance.

Un autre avantage est de proposer une préparation cosmétique se conservant pendant un long temps, dans un milieu stérile (emballage stérile et hermétique), ainsi que dans un milieu non stérile (emballage non hermétique ou après ouverture).

Le chitosane est obtenu par déacétylation de la chitine avec un alcali (hydroxyde de sodium ou de potassium, par exemple). La chitine est un des biopolymères les plus abondants sur terre. C'est un composant important de l'exosquelette des arthropodes, tel que la cuticule des insectes ou la carapace des crustacés, par exemple. La chitine est aussi un élément constitutif de la paroi cellulaire de certaines moisissures et levures.

On suppose que l'activité biologique du chitosane est due à sa nature polycationique. Les groupes amine du chitosane, lorsqu'ils sont protonés, entrent en interaction avec les sites électronégatifs se trouvant à la surface des cellules des microorganismes.

Cette interaction entraîne une modification de la perméabilité de la membrane cellulaire qui va provoquer la fuite vers l'extérieur de certains constituants intra-cellulaires ou de certains ions. Cette perte de substances intracellulaires est associée à l'inhibition de croissance du microorganisme. D'autres mécanismes générateurs d'énergie, tels que la respiration ou la fermentation peuvent également être perturbés et / ou bloqués par l'adjonction de chitosane.

Le chitosane utilisé selon la présente invention pourra être d'origine diverse. Il pourra être notamment le produit d'une déacétylation de chitine provenant de crabes, de crevettes ou de homards par exemple.

Les propriétés physico-chimiques et la microstructure du chitosane peuvent dépendre de la source choisie, ainsi que des différents traitements apportés, tels que la purification ou la déacétylation par exemple. Le chitosane préféré selon l'invention provient de chitine extraite de crevettes.

Le degré de déacétylation du chitosane peut exercer une certaine influence sur son activité biologique. Ce degré correspond à la quantité de groupes amine libérés, de sorte que plus il est important, plus le chitosane peut être efficace.

Le degré de déacétylation du chitosane selon la présente invention est de préférence compris entre 70 et 95%.

On a constaté que la longueur des chaines du polymère de chitosane peut influer sur son comportement et peut entrainer des modifications de son activité biologique, autrement dit de son pouvoir d'inhibition de la croissance des microorganismes.

On a constaté, en particulier, que lorsque le chitosane se présente sous forme d'un polymère de petite taille (monomère, pentamère, heptamère, par exemple) son activité biologique est très faible.

On suppose que ceci est dû au fait que la chaîne du polymère étant trop courte, la densité de charges locales est faible, et empêche le polymère d'interagir de manière souhaitable avec les sites négatifs à la surface de la membrane de la cellule. Il n'y aurait donc pas de modification notable de la perméabilité de cette membrane.

Les chitosanes de petite taille, donc de faible poids moléculaire, ne conviennent pas comme agent de conservation selon la présente invention.

Par faible poids moléculaire, on entend un poids inférieur à 3000.

On a constaté que les chitosanes de grande taille, donc à haut poids moléculaire possédent aussi une activité biologique faible. On suppose que cela est dû à une modification de la conformation moléculaire du chitosane. Le chitosane peut présenter une conformation allant d'un simple enroulement de nature aléatoire à une structure plus compacte, quasi globulaire, cette seconde structure étant probablement favorisée dans le cas des hauts poids moléculaires.

L'interaction électrostatique entre le chitosane et la surface des cellules des microorganismes pourrait alors être limitée, du fait que la plupart des groupes actifs du chitosane seraient confinés à l'intérieur de l'édifice moléculaire et stériquement inaccessibles. Les chitosanes de grande taille, donc de haut poids moléculaire, ne conviennent pas comme agent de conservation selon la présente invention.

Par haut poids moléculaire, on entend un poids supérieur à 700 000 .

Le chitosane capable d'inhiber la croissance des microorganismes selon la présente invention présente donc un poids moléculaire compris entre 3 000 et 700 000, et de préférence entre 120 000 et 450 000 .

La viscosité d'une solution de chitosane étant fonction de son poids moléculaire, cette grandeur s'est avérée utilisable pour caractériser une solution de chitosane utilisable dans le cadre de la présente invention.

C'est ainsi que dans un mode préféré d'utilisation du chitosane comme agent de conservation d'une préparation cosmétique, selon la présente invention, on incorpore le chitosane à la préparation cosmétique sous forme d'une solution aqueuse présentant une viscosité de 1 à 80 mPa.s pour une concentration de 1% de chitosane.

Le poids moléculaire du chitosane et la viscosité des solutions de chitosane sont mesurés selon les méthodes décrites à la fin de la partie générale de la description.

La teneur en chitosane de la préparation cosmétique peut influer sur l'activité biologique du chitosane. Une teneur trop élevée peut induire des phénomènes de répulsion entre polymères de chitosane, et empêcher ceux-ci de s'approcher de la membrane cellulaire.

Une teneur trop faible peut ne pas assurer une protection suffisante de la préparation vis-à-vis de la contamination microbienne.

La préparation cosmétique selon la présente invention peut présenter une teneur en chitosane comprise entre 50 et 5000 $\mu$g/g, et de préférence entre 100 et 300 $\mu$g/g.

L'activité biologique du chitosane peut aussi dépendre de sa solubilité. Le chitosane incorporé à une préparation cosmétique sous forme de poudre en suspension aqueuse peut être moins actif que sous forme de solution.

La solubilité du chitosane peut être améliorée par traitement aux ultrasons d'une dispersion de chitosane et/ou par lyophilisation après centrifugation. Le traitement aux ultrasons peut être un excellent moyen pour scinder les chaînes de polymères afin de les rendre plus solubles. Ceci amène un réarrangement de la microstructure du chitosane, qui permet d'augmenter sa solubilité et même son activité biologique. Cette activité peut être doublée, voire même multipliée par huit lors de l'utilisation de chitosane traité aux ultrasons et/ou lyophilisé.

De plus, pour être biologiquement actif, le chitosane doit être, de préférence, dans sa forme polycationique, c'est-à-dire à un pH inférieur à son pKa, qui est de 6,2.

Afin d'assurer une grande densité de charges positives, les solutions de chitosane ont préférentiellement un pH compris entre 5,0 et 5,5.

Le chitosane pourra être incorporé à toute préparation cosmétique, telle qu'un lait, une lotion, une crème ou un gel. Il est simplement préférable que la préparation cosmétique présente une faible teneur en anions.

Afin de tester l'activité biologique des différents chitosanes, on utilise un certain nombre de microorganismes.

3

Ces microorganismes sont choisis parmi ceux présentant un risque pour la santé des utilisateurs ou parmi ceux susceptibles d'entraîner une détérioration de la préparation cosmétique. Parmi les nombreux microorganismes répondant à ces critères, ont été choisies:
- des bactéries gram négatives (Escherichia coli et Pseudomonas aeruginosa),
- des bactéries gram positives (Staphylococcus aureus, Streptococcus faecalis et Bacillus cereus) et
- des levures (Candida albicans).

Méthodes de mesure

. Viscosité

La viscosité est mesurée sur des solutions à 1% de chitosane et 1% d'acide acétique, laissées au repos pendant 2 semaines afin que la viscosité se stabilise. La mesure est effectuée à l'aide d'un rhéomètre VOR Bohlin dans les conditions suivantes:

| | |
|---|---|
| système de mesure | cylindre C. 14 |
| barre de tension | 0,24 g.cm et 19,8 g.cm |
| temps d'attente initial | 60 s |
| temps de la mesure | 20 s à déformation constante |
| temps d'intégration | 10 s |
| température | 20°C |
| vitesse de cisaillement | 11,64 s$^{-1}$ |

. Poids moléculaire

Le poids moléculaire est mesuré par diffusion de lumière laser, selon la méthode de R.A. Muzzarelli, C. Lough et M. Emanuelli décrite dans l'article "The molecular weight of chitosans studied by laser light-scattering", publié dans Carbohydrate Res. 164, 433-442 (1987).

. Croissance des microorganismes

Le nombre de germes peut être déterminé de deux manières: par comptage ou par spectrophotométrie.
1) Comptage
Un échantillon de 1 ml est prélevé du milieu à analyser et dilué avec 9 ml d'une solution saline physiologique. On dilue de 10 en 10 cette solution, puis on place 1 ml de solution diluée sur une plaque de Pétri et on mélange avec 10 ml d'agar nutritif. On laisse incuber la plaque à 37°C pendant une nuit, puis on compte le nombre de germes qui se sont développés.
2) Spectrophotométrie
La méthode précédente présentant l'inconvénient d'être lente, il est plus aisé de mesurer le nombre de germes en suspension par spectrophotométrie. Une suspension de germes transmet la lumière la traversant d'une manière différente de celle d'une suspension témoin ne contenant pas de germes.
On mesure par spectrophotométrie la lumière transmise pour chaque solution, et la réduction de transmission permet de déterminer le nombre de germes en suspension. Cette mesure se fait à l'aide d'un spectrophotomètre, à une longueur d'onde de 550 nm.
Toutefois, cette méthode n'est pas très sensible et ne permet de détecter qu'un nombre de germes supérieur ou égal à $10^6$ germes par ml.
3) C.albicans
En ce qui concerne cette levure, on procède par comptage microscopique, à l'aide d'une cellule de Neubauer.
L'invention sera mieux comprise à la lecture des tests et exemples ci-après, les tests illustrant l'activité biologique in vitro de différents échantillons de chitosane. Les pourcentages sont donnés en poids, à l'exception du degré de déacétylation.

Tests d'activité biologique

Préparation d'échantillons de chitosane

On prépare différents échantillons de chitosane sous forme de milieux aqueux de culture de microorganismes contenant du chitosane en solution, de la manière suivante:

. pour les bactéries, on prépare un milieu aqueux de CAYE contenant 1% de caséine hydrolysée, 0,5% d'extrait de levure et 0,2% de glucose

. pour la levure, on prépare un milieu aqueux YES contenant 0,5% d'extrait de levure et 2% de sucrose

. on acidifie le milieu jusqu'à pH 2, par ajout d'acide chlorhydrique 1 N

. on dissout divers chitosanes de diverses origines, présentant tous un degré de déacétylation compris entre 80 et 95%, dans une solution à 1% d'acide chlorhydrique de manière à obtenir une concentration en chitosane de 1%

. on sépare le milieu de culture en plusieurs lots auxquels on ajoute, sous agitation, le chitosane

. le pH de chaque lot est ajusté à 5,8 par addition de soude 1 N, et le volume final est ajusté avec de l'eau distillée

. les différents lots sont stérilisés en autoclave à 121°C, pendant 15 min.

Les échantillons témoins ne contiennent que les milieux de culture dont le pH a été ajusté à 5,8.

Les différents chitosanes testés sont classés dans le tableau ci-après par ordre de poids moléculaire et de viscosité croissants:

| Echantillon | Poids moléculaire | Viscosité (mPa.s) |
|---|---|---|
| 1 | 127925 | 7,85 |
| 2 | 135190 | 7,59 |
| 3 | 166430 | 13,69 |
| 4 | 193610 | 53,02 |
| 5 | 233600 | 50,22 |
| 6 | 254050 | 27,69 |
| 7 | 318890 | 51,48 |
| 8 | 351460 | 64,03 |
| 9 | 356400 | 51,85 |
| 10 | 437710 | 69,99 |
| 11 | 781960 | 82,82 |
| 12 | 993530 | 120,0 |
| 13 | 1 140 000 | 172,7 |

Préparation de cultures de microorganismes

Les bactéries sont cultivées pendant 8 h sur un milieu nutritif (infusion à base de veau gélosée), puis 16 h sur un milieu de CAYE à pH 5,8.

La levure est cultivée pendant 3 jours sur un milieu YES à pH 5,8.

La culture est ensuite diluée de telle sorte que 1 ml de culture contienne approximativement $10^7$ germes.

Test No 1

Les différents échantillons de chitosane, dont la concentration est de 50 $\mu$g de chitosane par ml de milieu de culture, sont inoculés avec une suspension de germes de Candida albicans, de manière à obtenir un nombre initial de environ $10^5$ germes par ml.

On laisse incuber les échantillons à 23°C sous agitation à 110 tours/min.

On mesure quotidiennement le nombre de germes dans chaque échantillon.

On note une inhibition de la croissance de C. albicans due au chitosane. On mesure l'intervalle de temps séparant le jour de l'inoculation de C. albicans, et le jour où le nombre de germes de C. albicans recommence à croître.

On obtient les résultats suivants: (T = témoin)

| Echantillon | T | 1 | 2 | 3 | 4 | 5 | 6 | 7 | 8 | 9 | 10 | 11 | 12 | 13 |
|---|---|---|---|---|---|---|---|---|---|---|---|---|---|---|
| Intervalle de temps (jour) | 0 | 28 | 18 | 25 | 14 | 14 | 13 | 12 | 12 | 15 | 23 | 3 | 5 | 3 |

comparaison (11-12-13)

On remarque que l'ajout des échantillons de chitosane 1-10 permet de retarder la reprise de croissance du nombre de germes pendant une période d'au moins 12 jours.

Test No 2

Différents échantillons de chitosane, dont la concentration est de 100 $\mu$g de chitosane par ml de milieu de culture, sont inoculés avec des suspensions de différents organismes à raison d'un microorganisme par échantillon, de manière à obtenir un nombre initial de environ $10^5$ germes par ml.

On laisse incuber les échantillons à 37°C (43°C pour Ps. aeruginosa), et on note l'influence du chitosane sur la reprise de croissance des bactéries en mesurant, à intervalle régulier, le nombre de germes, par spectrophotométrie.

A titre d'illustration, le tableau ci-après donne, pour quatre échantillons de chitosane, l'évolution du pourcentage de transmission au cours de temps pour la bactérie Staph. aureus.

Le pourcentage de transmission est inversement proportionnel au nombre de germes en solution (0 signifiant que la lumière est difficilement transmise, vu le nombre très important des germes présents).

| Echantillon | % de transmission | | | | | |
|---|---|---|---|---|---|---|
| | 0h | 6h | 8h | 24h | 48h | 120h |
| 2 | 70 | 70 | 80 | 91 | 93 | 7 |
| 4 | 46 | 46 | 46 | 46 | 63 | 7 |
| 5 | 61 | 68 | 68 | 74 | 64 | 7 |
| 9 | 91 | 91 | 91 | 91 | 82 | 7 |
| Témoin | 100 | 70 | 25 | 7 | 7 | 7 |

Le pourcentage de transmission initial est différent selon les échantillons testés. Ceci est dû à l'apparition d'une légère turbidité dans la solution, due à une interaction entre le chitosane et le milieu de culture. Toutefois cette turbidité restant constante au cours du temps, elle n'influe pas sur le tracé général des courbes d'évolution.

Pour chaque bactérie testée, les échantillons de chitosane ont été classés en fonction de la durée de leur pouvoir d'inhibition.

- Staph. aureus
  . au moins 8 h: 1
  . au moins 24 h: 3-6-7-8-10
  . au moins 48 h: 2-4-5-9
- Strep. faecalis
  . au moins 6 h: 11-12 (comparaison)
  . au moins 8 h: 3-7-10
  . au moins 48 h: 2-4-5-6-8-9
- B. cereus
  . au moins 24 h: 6-7-8
  . au moins 96 h: 1-2-3-4-5-9-10
- E. coli
  . au moins 6 h: 9

. au moins 8 h: 2-4
- Ps. aeruginosa
  . au moins 10 h: 3-4-10
  . au moins 14 h: 2-5-9

On peut conclure de ce test que les échantillons de chitosane 1-10 testés inhibent de façon appréciable la croissance des bactéries.


Test No 3

On procède de manière semblable à celle décrite au test No 2, à l'exception du fait que l'on utilise des échantillons de chitosane dont la concentration est de 200 $\mu$g de chitosane par ml de milieu de culture et que l'on détermine le nombre de germes par comptage.

Les tableaux suivants montrent l'évolution du nombre de germes par ml au cours du temps, pour différents échantillons de chitosane et pour différents microorganismes.

ND = non déterminable
- Streptococcus faecalis
  Initialement: $2,6.10^6$ germes par ml

| Echantillon | Nombre de germes par ml | | | | |
|---|---|---|---|---|---|
| | 4h | 8h | 24h | 48h | 144h |
| 1 | $3,1.10^4$ | 2200 | $10^6$ | $>10^7$ | $5,6.10^6$ |
| 2 | 1800 | $4,6.10^4$ | $2.10^4$ | $66.10^4$ | $5,6.10^6$ |
| 3 | 93 | 26 | 120 | 290 | $5,5.10^6$ |
| 8 | 20 | 50 | $1,4.10^6$ | $>10^7$ | $58.10^4$ |
| Témoin | $45.10^6$ | $2.10^8$ | $2,8.10^8$ | ND | ND |

- Staphylococcus aureus
  Initialement: $8,1.10^6$ germes par ml

| Echantillon | Nombre de germes par ml | | | | | |
|---|---|---|---|---|---|---|
| | 4h | 8h | 24h | 48h | 72h | 168h |
| 1 | 6200 | 3100 | $55.10^4$ | $12.10^4$ | $49.10^4$ | $>10^7$ |
| 2 | 400 | 43 | $28.10^4$ | $2,4.10^6$ | $3.6.10^6$ | $>10^7$ |
| 3 | $12.10^4$ | 2 | $5,8.10^4$ | $12.10^4$ | $10^5$ | 6300 |
| 8 | 100 | 100 | $16.10^4$ | $17.10^4$ | $5,7.10^4$ | $9,2.10^4$ |
| Témoin | $17.10^6$ | $58.10^6$ | $10^8$ | ND | ND | ND |

- Bacillus cereus
  Initialement: $9,1.10^5$ germes par ml

| Echantillon | Nombre de germes par ml | | | | |
|---|---|---|---|---|---|
| | 4h | 8h | 24h | 48h | 120h |
| 1 | 7 | 4 | 0 | 0 | 0 |
| 2 | 0 | 0 | $39.10^4$ | $1,3.10^6$ | $2,1.10^4$ |
| 3 | 8 | 2 | 1 | 0 | 0 |
| 8 | 1 | 0 | 0 | 0 | $1,2.10^6$ |
| Témoin | $11.10^6$ | $3.10^7$ | $4.10^6$ | ND | ND |

- Pseudomonas aeruginosa
  Initialement: $8,1.10^6$ germes par ml

| Echantillon | Nombre de germes par ml | | | |
|---|---|---|---|---|
| | 4h | 8h | 24h | 48h |
| 1 | $27.10^4$ | $1,2.10^6$ | $>10^7$ | ND |
| 2 | 6200 | 1600 | 9800 | $2,1.10^6$ |
| 3 | 7300 | 3400 | $7,7.10^4$ | $>10^7$ |
| 8 | $34.10^4$ | $63.10^4$ | $>10^7$ | ND |
| Témoin | $19.10^6$ | $36.10^6$ | $>10^9$ | ND |

- E. coli
  Initialement: $1,6.10^5$ germes par ml

| Echantillon | Nombre de germes par ml | | |
|---|---|---|---|
| | 4h | 8h | 24h |
| 1 | 6600 | $1,2.10^6$ | $>10^8$ |
| 2 | $1,8.10^4$ | $>10^7$ | $>10^8$ |
| 3 | 6400 | $1,2.10^6$ | $>10^8$ |
| 8 | $36.10^4$ | $>10^7$ | $>10^8$ |
| Témoin | $6,3.10^6$ | $4.10^8$ | $2.10^9$ |

Durant les quatre ou même huit premières heures d'incubation, le nombre de germes présents dans les échantillons contenant du chitosane diminue de façon significative.

Ce nombre tend ensuite à retrouver sa valeur initiale, et même à la dépasser. Ceci montre que les échantillons contenant du chitosane ont une activité bactériostatique vis-à-vis des microorganismes testés, et ceci sur des périodes allant de 4 heures (E. coli) à 48 heures (St. faecalis et St. aureus).

On remarque aussi que les échantillons 1 et 3 ont une activité bactéricide vis-à-vis de la bactérie B. cereus.

Ces trois tests montrent bien que des échantillons de chitosane ayant un poids moléculaire compris entre 3000 et 700 000 ont une activité bactériostatique, voire bactéricide, vis-à-vis des microorganismes.

Test No 4

On prépare, à partir de chitosane ayant approximativement un poids moléculaire de 128 000, plusieurs échantillons de concentrations différentes, s'échelonnant de 50 g à 5000 μg de chitosane par ml de milieu.

Ces échantillons sont inoculés avec une suspension de C. albicans de manière à obtenir un nombre initial de environ $10^5$ germes par ml, puis chaque échantillon est réparti dans plusieurs tubes en verre, stériles qui sont ensuite fermés hermétiquement.

On laisse incuber à 23°C dans l'obscurité, pendant 6 mois. A intervalle de temps régulier, on ouvre un tube pour chaque concentration et l'on mesure le nombre de germes présents.

Pour chaque concentration, on obtient une durée pendant laquelle le chitosane est actif et inhibe la croissance des germes présents.

Pour les échantillons contenant 250 μg et plus de chitosane par ml, la croissance des germés est inhibée pendant une durée minimale de 6 mois. En effet, on ne détecte aucune croissance de germes dans ces tubes.

Pour l'échantillon contenant 50 μg de chitosane par ml, la croissance des germes est inhibée pendant 5 semaines, puis reprend ensuite.

A titre de comparaison, pour un échantillon contenant 20 μg de chitosane par ml, la croissance des germes n'est inhibée que pendant 1 jour.

Le chitosane peut donc rester actif pendant de longues périodes, s'il est en concentration suffisante.

Test No 5 (test comparatif)

Afin de pouvoir étudier l'activité des chitosanes de faible poids moléculaire, on hydrolyse partiellement 5 g de chitosane, avec HCl concentré, pendant 21 h à 53°C. Le chitosane résultant est séché sous vide à 30°C, et les différents oligomères sont séparés grâce à une résine échangeuse d'ions (AG 50 W - X 8), l'éluant utilisé étant l'acide chlorhydrique, de concentration variant de 1 à 5,5 N. Les différentes fractions d'éluat sont séparées par filtration sur gel (Fractogel TSK HW - 40) l'éluant utilisé étant une solution à 0,2% d'acide formique. Les différents oligomères sont identifiés par chromatographie sur couche mince.

On teste l'activité antimicrobienne du pentamère et de l'heptamère de glucosamine, selon la méthode décrite dans le test No 4, à l'aide de la levure C. albicans, et ce pour différentes concentrations en chitosane.

On ne remarque aucune inhibition de la croissance de la levure pour une concentration de 1 mg de chitosane par ml de milieu de culture (soit 1000 $\mu$g/g).

Exemple 1

Dans 600 ml d'eau distillée on disperse 10 g de chitosane présentant un poids moléculaire de environ 180000, un degré de déacétylation de environ 85% et provenant de crevettes. On ajoute 30 ml d'acide acétique, et on dissout le tout par agitation. On ajuste le pH à 5,8 par ajout de soude, et on ajuste le volume de la solution à 1 litre par addition d'eau distillée.

On prépare parallèlement quatre lots de lait de toilette, contenant 6% d'ester alkylique en $C_{12}$-$C_{18}$ de polyéthylène glycol, 5% de stéarate de glycérol, 3% de laurate d'isodécyl, 3% de triglycérides d'acides gras en $C_{10}$-$C_{18}$, 1% d'alcool cétylique, 0,8% de diméthicone, (huile de silicone), 0,4% d'hydroxyéthylcellulose, 3% de sorbitol à 70% et 0,2% de parfum.

On réserve un lot comme témoin et l'on ajoute à chacun des trois autres lots quelques ml de solution de chitosane de manière à obtenir quatre lots présentant des teneurs respectives en chitosane de 0, 50, 100 et 200 $\mu$g/g.

On ajuste le pH à 6 et l'on complète à 100% avec de l'eau distillée.

On inocule les lots avec une suspension de microorganismes de manière à obtenir un nombre initial de environ $10^4$ germes par ml.

Les lots sont conservés à température ambiante et à l'abri de la lumière.

On mesure le nombre de germes présents dans le lait de toilette à différents stades de l'évolution: après l'inoculation, après une semaine, après deux semaines, après trois semaines et après quatre semaines.

Les microorganismes choisis sont:

I. E. coli

II. Ps. aeruginosa

III. Staph. aureus

IV. Strep. faecalis

V. C. albicans

On obtient les résultats présentés dans le tableau suivant.

| Microorganismes | I | II | III | IV | V |
|---|---|---|---|---|---|
| Après inoculation | | | | | |
| Lot A (témoin) | $34.10^3$ | $8.10^4$ | $42.10^4$ | $7.10^4$ | $36.10$ |
| Lot B (50 $\mu$g/g) | $35.10^3$ | 10 | $18.10^3$ | $7.10^4$ | $9.10^3$ |
| Lot C (100 $\mu$g/g) | $29.10^3$ | 10 | $6.10^4$ | $53.10^3$ | $9.10^3$ |
| Lot D (200 $\mu$g/g) | $24.10^3$ | 10 | $27.10^3$ | $24.10^3$ | 80 |
| Après 1 semaine | | | | | |
| Lot A | $3.10^7$ | $16.10^5$ | $10^4$ | 3500 | $3.10^6$ |
| Lot B | 10 | 10 | 10 | 10 | 10 |
| Lot C | 10 | 10 | 10 | 10 | 10 |
| Lot D | 10 | 10 | 10 | 10 | 10 |
| Après 2 semaines | | | | | |
| Lot A | $13.10^6$ | $10^6$ | 500 | 20 | $10^6$ |
| Lot B | - | - | - | - | 10 |
| Lot C | - | - | - | - | 10 |
| Lot D | - | - | - | - | 10 |
| Après 3 semaines | | | | | |
| Lot A | $6.10^6$ | $48.10^5$ | 60 | 50 | $8.10^6$ |
| Lot B | - | - | - | - | 10 |
| Lot C | - | - | - | - | 10 |
| Lot D | - | - | - | - | 10 |
| Après 4 semaines | | | | | |
| Lot A | $5.10^6$ | $10^7$ | 50 | 10 | $9.10^6$ |
| Lot B | - | - | - | - | 10 |
| Lot C | - | - | - | - | 10 |
| Lot D | - | - | - | - | 10 |

On remarque que tous les laits contenant du chitosane inhibent la croissance des bactéries ou levures qu'ils contiennent. L'effet est visible dès la première semaine.

L'activité bactériostatique voire bactéricide du chitosane est nette, même en ce qui concerne les laits à faible teneur en chitosane (50 $\mu$g/g).

Exemple 2

Une crème cosmétique contenant du chitosane est préparée de la manière suivante:
- on prépare 77 ml d'une solution tampon à pH 5,7 (à base de phosphate de sodium),
- on y ajoute 10 mg de chitosane, dissout dans de l'acide acétique dilué, présentant un poids moléculaire de environ 180000, un degré de déacétylation de environ 85% et provenant de crevettes,
- on ajoute ensuite 8 g de glycérol, puis le mélange est chauffé au bain-marie à 80°C,
- parallèlement, on fait fondre 15 g de stéarate de glycérol à 80°C, qu'on ajoute lentement au mélange précédent, sous agitation,
- on laisse le mélange se refroidir jusqu'à température ambiante, en l'homogénéisant de temps à autre.

On obtient une crème contenant 100 $\mu$g/g de chitosane. De la même manière, on prépare une crème contenant 250 $\mu$g/g de chitosane.

Des crèmes témoins, sans ajout de chitosane, sont aussi préparées.

Les différentes crèmes sont placées dans des pots non stérilisés, et gardées à température ambiante, dans l'obscurité. Des signes de contamination apparaissent au bout de 2 mois sur les témoins. Un examen microscopique révèle l'apparition graduelle de spores et d'hyphes de champignons.

Les crèmes contenant du chitosane ne présentent aucune trace de contamination, même au bout d'un an.

**Revendications**

1. Préparation cosmétique contenant du chitosane, caractérisée en ce que le chitosane a un poids moléculaire compris entre 3000 et 700 000.

2. Préparation cosmétique selon la revendication 1, caractérisée en ce que le chitosane a un poids moléculaire compris entre 120000 et 450000.

3. Préparation cosmétique selon la revendication 1, caractérisée en ce que le chitosane a un degré de déacétylation compris entre 70 et 95%.

4. Préparation cosmétique selon la revendication 1, caractérisée en ce que sa teneur en chitosane est comprise entre 50 et 5000 $\mu$g/g.

5. Préparation cosmétique selon la revendication 1, caractérisée en ce que le chitosane provient de la crevette.

6. Utilisation du chitosane comme agent de conservation d'une préparation cosmétique, caractérisée par le fait que l'on incorpore à la préparation cosmétique un chitosane présentant un poids moléculaire compris entre 3000 et 700 000.

7. Utilisation selon la revendication 6, caractérisée par le fait que l'on incorpore un chitosane présentant un poids moléculaire compris entre 120000 et 450000.

8. Utilisation selon la revendication 6, caractérisée par le fait que l'on incorpore un chitosane présentant un degré de déacétylation compris entre 70 et 95%.

9. Utilisation selon la revendication 6, caractérisée par le fait que l'on incorpore 50 à 5000 $\mu$g de chitosane par g de préparation cosmétique.

10. Utilisation selon la revendication 6, caractérisée par le fait que l'on incorpore le chitosane sous forme d'une solution aqueuse présentant un pH inférieur à 6,2.

11. Utilisation selon la revendication 10, caractérisée par le fait que l'on incorpore le chitosane sous forme d'une solution aqueuse présentant un pH de 5,0-5,5.

12. Utilisation selon la revendication 6, caractérisée par le fait que l'on incorpore le chitosane sous forme d'une solution aqueuse contenant 1% de chitosane et présentant une viscosité comprise entre 1 et 80 mPa.s.

13. Utilisation selon la revendication 6, caractérisée par le fait que l'on incorpore le chitosane sous forme d'une poudre en suspension aqueuse.

14. Utilisation selon la revendication 6, caractérisée par le fait que l'on incorpore le chitosane sous forme d'une poudre obtenue par mise en dispersion aqueuse du chitosane, traitement aux ultrasons de la dispersion, centrifugation et lyophilisation.

**Claims**

1. A cosmetic preparation containing chitosan, characterized in that the chitosan has a molecular weight in the range from 3,000 to 700,000.

2. A cosmetic preparation as claimed in claim 1, characterized in that the chitosan has a molecular weight in the range from 120,000 to 450,000.

3. A cosmetic preparation as claimed in claim 1, characterized in that the chitosan has a degree of deacetylation of from 70 to 95%.

4. A cosmetic preparation as claimed in claim 1, characterized in that it has a chitosan content of from 50 to 5,000 $\mu$g/g.

5. A cosmetic preparation as claimed in claim 1, characterized in that the chitosan comes from shrimps.

6. The use of the chitosan as a preservative in a cosmetic preparation, characterized in that a chitosan having a molecular weight in the range from 3,000 to 700,000 is incorporated in the cosmetic preparation.

7. The use claimed in claim 6, characterized in that a chitosan having a molecular weight in the range from 120,000 to 450,000 is incorporated.

8. The use claimed in claim 6, characterized in that a chitosan having a degree of deacetylation of from 70 to 95% is incorporated.

9. The use claimed in claim 6, characterized in that from 50 to 5,000 $\mu$g chitosan are incorporated per g cosmetic preparation.

10. The use claimed in claim 6, characterized in that the chitosan is incorporated in the form of an aqueous solution having a pH below 6.2.

11. The use claimed in claim 10, characterized in that the chitosan is incorporated in the form of an aqueous solution having a pH of 5.0 to 5.5.

12. The use claimed in claim 6, characterized in that the chitosan is incorporated in the form of an aqueous solution containing 1% chitosan and having a viscosity in the range from 1 to 80 mPa.s

13. The use claimed in claim 6, characterized in that the chitosan is incorporated in the form of a powder in aqueous suspension.

14. The use claimed in claim 6, characterized in that the chitosan is incorporated in the form of a powder obtained by dispersion of the chitosan in water, ultrasonication of the dispersion, centrifugation and freeze-drying.

**Patentansprüche**

1. Chitosanhaltiges kosmetisches Präparat, dadurch gekennzeichnet, daß das Chitosan ein Molekulargewicht von 3000 bis 700.000 hat.

2. Kosmetisches Präparat nach Anspruch 1, dadurch gekennzeichnet, daß das Chitosan ein Molekulargewicht von 120.000 bis 450.000 hat.

3. Kosmetisches Präparat nach Anspruch 1, dadurch gekennzeichnet, daß das Chitosan einen Deacetylierungsgrad von 70 bis 95% hat.

4. Kosmetisches Präparat nach Anspruch 1, dadurch gekennzeichnet, daß sein Gehalt an Chitosan 50 bis 5.000 $\mu$g/g beträgt.

5. Kosmetisches Präparat nach Anspruch 1, dadurch gekennzeichnet, daß das Chitosan von Garnelen stammt.

6. Verwendung von Chitosan als Konservierungsmittel für ein kosmetisches Präparat, dadurch gekennzeichnet, daß dem kosmetischen Präparat ein Chitosan mit einem Molekulargewicht von 3.000 bis 700.000 beigegeben wird.

7. Verwendung nach Anspruch 6, dadurch gekennzeichnet, daß ein Chitosan mit einem Molekulargewicht von 120.000 bis 450.000 beigegeben wird.

**8.** Verwendung nach Anspruch 6, dadurch gekennzeichnet, daß ein Chitosan mit einem Deacetylierungsgrad von 70 bis 95% beigegeben wird.

**9.** Verwendung nach Anspruch 6, dadurch gekennzeichnet, daß 50 bis 5.000 $\mu$g Chitosan pro g kosmetisches Präparat beigegeben wird.

**10.** Verwendung nach Anspruch 6, dadurch gekennzeichnet, daß das Chitosan in Form einer wässrigen Lösung mit einem pH-Wert von unter 6,2 beigegeben wird.

**11.** Verwendung nach Anspruch 10, dadurch gekennzeichnet, daß das Chitosan in Form einer wässrigen Lösung mit einem ph-Wert von 5,0 bis 5,5 beigegeben wird.

**12.** Verwendung nach Anspruch 6, dadurch gekennzeichnet, daß das Chitosan in Form einer wässrigen Lösung mit einem Chitosangehalt von 1% und einer Viskosität von 1 bis 80 mPa.s beigegeben wird.

**13.** Verwendung nach Anspruch 6, dadurch gekennzeichnet, daß das Chitosan in Form eines Pulvers in wässriger Lösung beigegeben wird.

**14.** Verwendung nach Anspruch 6, dadurch gekennzeichnet, daß das Chitosan in Form eines Pulvers beigegeben wird, das durch Dispergierung des Chitosans in Wasser, Behandlung der Dispersion mit Ultraschall, Zentrifugierung und Lyophilisierung hergestellt wird.